# EUROPEAN PATENT SPECIFICATION

(11) **EP 1 545 463 B1**
(45) Date of publication and mention of the grant of the patent: **21.12.2011**
(21) Application number: 03766858.9
(22) Date of filing: 14.07.2003
(51) Int. Cl.: A61K 9/14, B01D 9/00

(54) **CRYSTALLINE DRUG PARTICLES PREPARED USING A CONTROLLED PRECIPITATION (RECRYSTALLIZATION) PROCESS**
KRISTALLINE ARZNEIMITTELTEILCHEN, DIE IN EINEM VERFAHREN MIT KONTROLLIERTER ABSCHEIDUNG (REKRISTALLISATION) HERGESTELLT WERDEN
PARTICULES MEDICAMENTEUSES CRISTALLINES OBTENUES PAR PROCEDE DE PRECIPITATION REGULEE (RECRISTALLISATION)

(30) Priority: 06.08.2002 US 213907
(43) Date of publication of application: 29.06.2005
(73) Proprietor: Board of Regents of the University of Texas System, Austin, TX 78701 (US)
(72) Inventor: SVENSON, Sonke, Midland, MI 48642 (US); TUCKER, Christopher, J., Midland, MI 48640 (US); HITT, James, E., Midland, MI 48642 (US)
(74) Representative: Weickmann, Heinrich
(86) International application number: PCT/US2003/021882
(87) International publication number: WO 2004/012710

(56) References cited:
- WO-A-03/033097
- US-A- 5 562 923
- US-A- 5 716 642
- POZARNSKY G A ET AL: "Preparation of monodisperse colloids of biologically active compounds 1. Naproxen" COLLOIDS AND SURFACES A: PHYSICOCHEMICAL AND ENGINEERING ASPECTS 1997 NETHERLANDS, vol. 125, no. 1, 1997, pages 47-52, XP002260310 ISSN: 0927-7757
- RUCH F ET AL: "Preparation of micrometer size budesonide particles by precipitation" JOURNAL OF COLLOID AND INTERFACE SCIENCE, ACADEMIC PRESS, NEW YORK, NY, US, vol. 229, no. 1, 1 September 2000 (2000-09-01), pages 207-211, XP002253141 ISSN: 0021-9797
- GOIA C ET AL: "Precipitation of barium and calcium naproxenate particles of different morphologies" JOURNAL OF COLLOID AND INTERFACE SCIENCE 15 OCT 1998 UNITED STATES, vol. 206, no. 2, 15 October 1998 (1998-10-15), pages 583-591, XP002260311 ISSN: 0021-9797
- QIPING ZHONG ET AL: "PREPARATION OF UNIFORM ZINC OXIDE COLLOIDS BY CONTROLLED DOUBLE-JETPRECIPITATION", JOURNAL OF MATERIALS CHEMISTRY, THE ROYAL SOCIETY OF CHEMISTRY, CAMBRIDGE, GB, vol. 6, no.3, 1 March 1996 (1996-03-01), pages 443-447, XP000589547, ISSN: 0959-9428, DOI:DOI:10.1039/JM9960600443
- QIPING ZHONG ET AL: "PREPARATION OF UNIFORM ZINC OXIDE COLLOIDS BY CONTROLLED DOUBLE-JETPRECIPITATION", JOURNAL OF MATERIALS CHEMISTRY, THE ROYAL SOCIETY OF CHEMISTRY, CAMBRIDGE, GB, vol. 6, no. 3, 1 March 1996 (1996-03-01), pages 443-447, XP000589547, ISSN: 0959-9428, DOI: DOI:10.1039/JM9960600443

## Description

The present invention relates to crystalline drug particles and in particular relates to crystalline drug particles prepared using a controlled precipitation process.

High bioavailability and dissolution rates are desirable attributes of a pharmaceutical end product. Bioavailability is a term meaning the degree to which a pharmaceutical product, or drug, becomes available to the target tissue after being administered to the body. Poor bioavailability is a significant problem encountered in the development of pharmaceutical compositions, particularly those containing an active ingredient that is poorly soluble in water. Poorly water soluble drugs tend to be eliminated from the gastrointestinal tract before being absorbed into the circulation.

It is known that the rate of dissolution of a particulate drug can increase with increasing surface area, such as by decreasing particle size. Furthermore, crystalline drug particles are desirable because of the greater stability as opposed to amorphous particles. Efforts have been made to control the size and morphology of drug particles in pharmaceutical compositions. The most commonly employed techniques are precipitation and milling techniques.

U.S. Patent 5,716,642 teaches the use of an acid-base precipitation method. However, the method described in the '642 patent results in a large concentration of salt which must be removed via dialysis in order to obtain relatively pure drug particles.

Examples of solvent precipitation methods are described in U.S. Patent Nos. 4,826.689 and 6,221,398 B1, in Hasegawa et al, "Supersaturation Mechanism of Drugs from Solid Dispersions with Enteric Coating Agents, Chem. Pharm. Bull. Vol. 36, No. 12, p. 4941 (1988), and Frederic Ruch and Egon Matijevic, Preparation of Micrometer Size Budesonide Particles by Precipitation, Journal of Colloid and Interface Science, 229, 207-211 (2000). In the standard method described in these references, a solution of the compound to be crystallized is contacted with an appropriate 'anti-solvent' in a stirred vessel. Within the stirred vessel, the anti-solvent initiates primary nucleation which leads to crystal formation. However, the crystals that are formed are relatively large, whereas the smaller particles described by these references are amorphous. For the relatively large crystalline particles, these methods almost always require a post-crystallization milling step in order to increase particle surface area and thereby improve their bioavailability. However, milling has drawbacks, including yield loss, noise and dust. Even wet milling techniques, as described in U.S. Patent No. 5,145,684, exhibit problems associated with contamination from the grinding media. Moreover, exposing a drug substance to excessive mechanical shear or exceedingly high temperatures can cause the drug to lose its activity or transform, at least in part, from the crystalline to the amorphous state, as described by Florence et al, Effect of Particle Size Reduction on Digoxin Crystal Properties, Journal of Pharmaceutics and Pharmacology, Vol. 26, No. 6, 479-480 (1974), and R. Suryanarayanan and A.G. Mitchell, Evaluation of Two Concepts of Crystallinity Using Calcium Gluceptate as a Model Compound, International Journal of Pharmaceutics, Vol. 24, 1-17 (1985). In addition, wet milling techniques always result in the presence of a fraction of larger particles, which affects the time for the particles to completely dissolve.

The crystal lattice is generally recognized to be a highly ordered structure which repeats itself regularly in three dimensions. However, as discussed in H. M. Burt and A. G. Mitchell, Crystal Defects and Dissolution, International Journal of Pharmaceutics, Vol. 9, 137-152 (1981), crystal lattice imperfections developed during the crystal growth step may cause dislocations within the crystal which are considered to be thermodynamically instable, resulting in an increase in free energy and a reduction in the activation energy for dissolution at points where the dislocations emerge on the crystal surface.

It is known that the presence of additives and impurities in solution may alter the morphology of crystals that are being formed from this solution, as described by Davey et al, Structural and Kinetic Features of Crystal Growth Inhibition: Adipic Acid Growing in the Presence of n-Alkanoic Acids, Journal of the Chemical Society, Faraday Transactions. Vol. 88(23), 3461-3466 (1992), and may influence the equilibrium between crystallization and dissolution of crystals in a pharmaceutical suspension formulation, as described by K. H. Ziller and H. Rupprecht, Control of Crystal Growth in Drug Suspensions, Drug Development and Industrial Pharmacy, Vol. 14(15-17), 2341-2370 (1988). However, the Davey and Ziller references focus on reducing the presence of dislocations in the crystal lattice in order to solve a perceived problem caused by such dislocations. The Davey and Ziller references do not address introducing such dislocations to control dissolution rates.

It would be an advantage in the art of preparation drug particles to provide particles which exhibit enhanced dissolution rates as compared with particles prepared according to methods described in the above prior art. It would also be an advantage if such particles were essentially crystalline in nature so as to minimize some of the problems associated with reduced stability of amorphous particles.

In one aspect, the present invention is a process for the preparation of particles comprising a plurality of crystalline domains, wherein each crystalline domain is oriented differently than any of the adjacent domains; and a plurality of interfacial regions surrounding the crystalline domains; wherein the crystalline domains comprise a drug substance, and wherein the interfacial regions comprise at least one stabilizer.

In a second aspect, the present invention is a process comprising the steps of: (a) dissolving a drug substance in a solvent; and (b) adding the product of step (a) to water to form precipitated drug particles; wherein the drug particles comprise: a plurality of crystalline domains, each domain being oriented differently than any of the adjacent domains, wherein the domains comprise a drug substance; and a plurality of interfacial regions surrounding the crystalline domains, the interfacial regions comprising at least one stabilizer.

The particles exhibit relatively fast dissolution times as compared to particles prepared by processes described in the prior art.

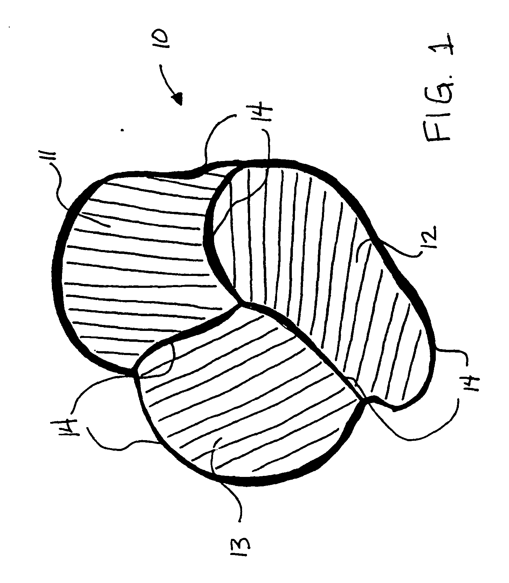

Figure 1 is an enlarged cross-sectional view of a particle of the present invention.

Figure 1 illustrates one embodiment of a particle 10 of the present invention. Particle 10 comprises a plurality of crystalline domains 11, 12, and 13. In Figure 1, the lines in each of the crystalline domains 11, 12, and 13 depict the orientation of the crystal lattice within each of the domains. As shown, the orientation of the crystal lattice in crystalline domain 11 is in a different direction than the orientation of the crystal lattice in crystalline domain 12, and the orientation of the crystal lattice in crystalline domain 11 is also in a different direction than the orientation of the crystal lattice in crystalline domain 13. Each crystalline domain is oriented differently than any of the adjacent domains.

The crystalline domains comprise a drug substance. In one embodiment, the drug substance is poorly soluble in water. Suitable drug substances can be selected from a variety of known classes of drugs including, for example, analgesics, anti-inflammatory agents, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytic sedatives (hypnotics and neuroleptics), astringents, beta-adrenoceptor blocking agents, blood products and substitutes, cardiacinotropic agents, contrast media, corticosterioids, cough suppressants (expectorants and mucolytics), diagnostic agents, diagnostic imaging agents, diuretics, dopaminergics (antiparkinsonian agents), haemostatics, immuriological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasidilators and xanthines. Preferred drug substances include those intended for oral administration. A description of these classes of drugs and a listing of species within each class can be found in Martindale, The Extra Pharmacopoeia, Twenty-ninth Edition, The Pharmaceutical Press, London, 1989.

The crystalline domains are preferably less than 500 Angstroms in size. More preferably, the crystalline domains are less than 450 Angstroms, and even more preferably less than 400 Angstroms.

A plurality of interfacial regions 14 surround the crystalline domains 11, 12, and 13. As shown in Figure 1, the interfacial regions 14 are in between each of the crystalline domains 11, 12, and 13, and the interfacial regions 14 are also on the outside surface of the particle 10.

The interfacial regions 14 comprise at least one stabilizer. The stabilizer should be chosen so as to reduce crystal growth so the crystalline domain size stays relative small and so that big crystals do not result. However, the stabilizer should also be chosen such that crystal growth is not prevented altogether, in order to ensure that some stabilizer is incorporated into the interfacial regions 14. Moreover, the stabilizer should be chosen so as not to prevent crystallization altogether, resulting in supersaturated solutions of the drug molecules. While not wishing to be bound by theory, incorporation of the stabilizer into the interfacial regions is what causes the particles of the present invention to exhibit relatively fast dissolution times.

The choice of stabilizer or stabilizers will depend upon the drug molecule. Generally, polymeric stabilizers are preferred. Examples of particle stabilizers include phospholipids, surfactants, polymeric surfactants, vesicles, polymers, including copolymers and homopolymers and biopolymers, and/or dispersion aids. Suitable surfactants include gelatin, casein, lecithin, phosphatides, gum acacia, cholesterol, tragacanth, fatty acids and fatty acid salts, benzalkonium chloride, glycerol mono and di fatty acid esters and ethers, cetostearyl alcohol, cetomacrogol 1000, polyoxyethylene castor oil derivatives, polyoxyethylene sorbitan fatty acid esters, for example, the commercially available Tweens, polyethylene glycols, poly(ethylene oxide/propylene oxide) copolymers, for example, the commercially available Poloxomers or Pluronics, polyoxyethylene fatty acid ethers, for example, the commercially available Brijs, polyoxyethylene fatty acid esters, sorbitan fatty acid esters, for example, the commercially available Spans, colloidal silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethylcellulose, noncrystalline cellulose, magnesium aluminum silicate, triethanolamine, polyvinyl alcohol (PVA), sodium lauryl sulfate, polyvinylpyrrolidone (PVP), poly(acrylic acid), and other anionic, cationic, zwitterionc and nonionic surfactants. Other suitable stabilizers are described in detail in the Handbook of Pharmaceutical Excipients, published jointly by the American Pharmaceutical Association and The Pharmaceutical Society of Great Britain, the Pharmaceutical Press, 1986. Such stabilizers are commercially available and/or can be prepared by techniques known in the art.

The drug particles prepared by the process of the present invention are essentially crystalline. As used herein the term "essentially crystalline" is defined to mean that the particles are at least 90% crystalline as measured using X-ray diffraction techniques.

The size of particle 10 as determined by light scattering techniques is not critical. In a preferred embodiment, however, the particles of the present invention are relatively small. More preferably, the particles of the present invention have a mean particle size of less than 20 microns, even more preferably less than 10 microns, and yet even more preferably less than 5 microns.

The particles prepared by the process of the present invention exhibit relatively fast dissolution rates. The preferred method for measuring dissolution rates for the particles of the present invention is a turbidity method. Turbidity gives a quantitative measurement of the change of intensity of light passing through a suspension of drug particles, caused by absorptive interactions resulting in energy transfer to the drug particles and by scattering from optical inhomogeneities in the drug particles. "Absorbance" is also a term that is used interchangeably with turbidity.

The turbidity method useful for determining the percent of dissolved material for the particles of the present invention comprises the following steps: determining the initial concentration of drug particles suspended in a liquid medium (i); determining the dynamic solid concentration (d) of drug particles in liquid medium; and calculating the percent dissolved material according to the formula: [(i-d)/i] x 100. Turbidity measurements are used to determine (i) and (d).

Any liquid medium can be used to measure turbidity, so long as the liquid medium is transparent in visible light and has a sufficiently different refractive index from the solid material such that it scatters light. The liquid medium should be chosen such that the equilibrium solubility of the drug particles in the liquid medium is between 5 and 500 mg/L. The term "equilibrium solubility" is defined herein to mean the maximum amount of drug particles that can be completely dissolved within 120 minutes in the liquid medium using this technique. To determine dissolution rates using turbidity measurements, one would need to develop a calibration curve showing turbidity versus a known concentration for the particular drug particles used. One would then measure the turbidity of the drug particles to be tested over time as the drug particles dissolve in the liquid medium, using commonly available light scattering equipment such as a colorimeter. One would then calculate i and d from the calibration curve based upon a measurement of turbidity. Such a method for measuring dissolution rates using turbidity is described in more detail in our copending U.S. application filed concurrently herewith.

When added to a liquid medium at a concentration that is from 25-95% of the equilibrium solubility, the particles of the present invention demonstrate complete dissolution in less than 5 minutes, as measured by the turbidity technique described above. The term equilibrium solubility is described above. More preferably, the drug particles can be added to the liquid medium at a concentration that is from 40-80% of their equilibrium solubility and still maintain complete dissolution in less than 5 minutes. As used herein, the term "complete dissolution" means that 95% of the particles are dissolved, as demonstrated by a 95% reduction in turbidity.

In one aspect of the present invention, the particles are prepared by way of a controlled precipitation process. A "controlled precipitation process" is defined herein to mean a process comprising the following steps: (a) dissolving a drug substance in a solvent; and (b) adding the product of step (a) to water to form precipitated drug particles. In a preferred embodiment, a stabilizer, such as those described above, is present in the solvent, in the water or in both the solvent and the water.

The solvent into which the drug is dissolved in step (a) can be any organic solvent or water/organic solvent blend which dissolves the drug adequately. Generally, the higher the solubility of the drug in the solvent, the more efficient the process will be. The solvent should be miscible in water. Preferably, the selected solvent exhibits ideal mixing behavior with water so that the solution can be instantaneously distributed throughout the water when added to the water in step (b). Suitable organic solvents include but are not limited to methanol, ethanol, isopropanol, 1-butanol, t-butanol, trifluoroethanol, polyhydric alcohols such as propylene glycol, PEG 400, and 1,3-propanediol, amides such as n-methyl pyrrolidone, N,N-dimethylformamide, tetrahydrofuran, propionaldehyde, acetone, n-propylamine, isopropylamine, ethylene diamine, acetonitrile, methyl ethyl ketone, acetic acid, formic acid, dimethylsulfoxide, 1,3-dioxolane, hexafluoroisopropanol, and combinations thereof.

The concentration of drug dissolved in the solvent in step (a) is preferably as close as practical to the solubility limit of the solvent at room temperature. Such concentration will depend upon the selected drug and solvent but is typically in the range of from 0.1 to 20.0 weight percent.

Optionally, one or more excipients are added to the solvent, to the water, or to both the solvent and the water. An excipient is defined herein as meaning something that changes the crystallization behavior of the molecules but is not incorporated into the resulting particles. Suitable excipients include organics, inorganics, acids, bases, salts, or mixtures thereof. Other suitable excipients are described in detail in the Handbook of Pharmaceutical Excipients, published jointly by the American Pharmaceutical Association and The Pharmaceutical Society of Great Britain, the Pharmaceutical Press, 1986. Such excipients are commercially available and/or can be prepared by techniques known in the art.

In a preferred embodiment, the controlled precipitation process further comprises the step of mixing the product of step (b). Any external device which imparts intense mixing of the drug/solvent in the water can be used. "Intense mixing" is defined herein as meaning that a uniformly supersaturated mixture is formed prior to particle nucleation. The mixing should be sufficiently intense so as to result in nearly instantaneous dispersion of the drug/solvent solution across the water before new particle growth occurs. Such intense mixing results in supersaturation of the drug substance in the solvent and liquid mixture, causing drug particles to precipitate into small particles having a crystalline structure. Examples of devices which may be used to mix the product of step (b) include a stir bar, and agitator, a homogenizer, and a colloid mill.

Optionally, the controlled precipitation process further comprises the step of recovering the precipitated drug particles. In one embodiment, recovering the drug particles comprises removing the solvent first and then subsequently removing the water. Alternatively, the solvent and water can be removed simultaneously from the particles. The choice will depend upon the concentration of solvent and the chosen method to remove the water. Removing the solvent can be performed using any desirable means including evaporation, dialysis and the like. Removing the water can be performed using any desirable means, including spray drying, spray freezing, gellation, (defined as gelling the particles with a polymer), lyophilization, or filtration.

As the drug in organic solvent is added to the water in step (b), the temperature of the product of step (b) is optimally controlled at a reduced temperature. Preferably, the temperature is controlled at less than 65 °C, more preferably less than 30 °C, even more preferably less than 23 °C, and most preferably less than 10 °C. The lower limit of the temperature of the dispersion is the freezing point of water. Temperatures which are too high could lead to undesirable particle growth.

### Examples

The following materials were used in the following examples:
"F-68", "F-77". F-88", F-108, and "F-127" means Pluronic^{®} polyethylene oxide/polypropylene oxide (EOₓ-PO_{y}-EOₓ) copolymers of different x:y ratios.
"Span 20" means sorbitan monolaurate.
"Tween 20" means polyoxyethylene 20 sorbitan monolaurate.
"PEG 150-C18" means polyoxyethylene 150 monostearate.
"PEG 150-diC18" means polyoxyethylene 150 distearate.
"PVP" means polyvinylpyrrolidone.
"PVA" means polyvinyl alcohol.
DCNa means deoxycholic acid sodium salt.

### Examples I through 21: Drug particles prepared using a controlled precipitation process.

0.3 g of the drug was dissolved in 6 ml of the organic solvent, which may contain 0.3 g of a stabilizer. The organic solution was injected at 2 degrees C with vigorous stirring into 30.0 g of the aqueous phase, which may contain a second stabilizer. The solvent was stripped from the resulting slurry, and the slurry freeze dried to yield a powder. For each example, X-ray diffraction patterns indicated that all samples were essentially crystalline. Average size of the crystalline domains was determined using X-ray diffraction as known by those skilled in the art of particle size measurement, using Jade XRD pattern processing software (v.6).

For each example containing the drug Danazol, to determine the time for complete dissolution, the following procedure utilizing turbidity measurements is followed. 5.1 mg of each sample was added to 150 ml of deionized water in a 200-ml plastic beaker equipped with a stir bar and a fiber optic turbidity probe (Brinkmann Colorimeter model PC-910 with a 650 nm light source filter and a 2 cm light path). The solubility of Danazol in water is approximately I mg per liter so one would expect approximately 0.22 mg of each sample to dissolve in 150 ml of water. After dispersing the sample for 150 seconds at a high stir rate, the stir speed was turned down to 100 rpm and 2.25 g of 20 % sodium dodecyl sulfate was added. The addition of this amount of sodium dodecyl sulfate raises the equilibrium solubility of Danazol to approximately 45 mg/l. At this level the amount of each sample present correspond to 50.3 % of the maximum amount of Danazol soluble in this media. Dissolution was then monitored by the loss in turbidity. The time to completely dissolve was the point at which there was a 95% reduction in the turbidity measurement.

For each example containing the drug Naproxen, to determine the time for complete dissolution, the following procedure utilizing turbidity measurements is followed. 6.5 mg of each sample was first dispersed by vortexing in 0.5 ml of deionized water for 40 seconds and then added to 150 ml of an aqueous sodium acetate/acetic acid buffer solution at pH 4.8. Dissolution was again monitored by the loss in turbidity. The time to completely dissolve was the point at which there was a 95% reduction in the turbidity measurement.

Each sample containing the drug Carbamazepine was treated in the same way as the samples containing Naproxen, except that deionized water was used instead of the sodium acetate/acetic acid buffer solution. Dissolution was monitored by the loss in turbidity. The time to completely dissolve was the point at which there was a 95% reduction in the turbidity measurement. Table A below lists the materials used and the results.

**Table A**

| **Ex.** | **Drug** | **Organic solution** | **Aqueous solution** | **Crystalline** | **Time to** | |
|---|---|---|---|---|---|---|
| | | | | **Domain** | **Complete** | |
| | | | | **[Å]** | **dissoln [s]** | |
| 1 | Naproxen | Methanol | 1.25% PVP 55KD/DCNa | 311 | 31 | |
| 2 | Naproxen | Methanol | 1.25% PVA 31-50KD/DCNa | 268 | 77 | |
| 3 | Naproxen | F-88, methanol | 1.25 wt% PVP 55KD | 319 | 16 | |
| 4 | Naproxen | F-88, methanol | 2.5 wt% PVP 55KD | 341 | 5 | |
| 5 | Naproxen | Span 20, methanol | 1.25 wt% PVP 55KD | 400 | 99 | |
| 6 | Naproxen | F-77, acetone | 1.25 wt% PVP 55KD | 333 | 46 | |
| 7 | Naproxen | F-108, acetone | 2.5 wt% PVA 31-50KD | 316 | 17 | |
| 8 | Naproxen | F-127, acetic acid | 1.25 wt% PVP 55KD | 391 | 15 | |
| 9 | Naproxen | F-88, acetic acid | 1.25 wt% PVP 55KD | 393 | 8 | |
| 10 | Carbamazepine | F-127, methanol | 1.25 wt% PVP 55KD | 313 | 20 | |
| 11 | Carbamazepine | PEG 150-C18, methanol | 1.25 wt% PVP 55KD | 325 | 36 | |
| 12 | Danazol | F-77, methanol | 1.25 wt% PVP 55KD | 320 | 19 | |
| 13 | Danazol | PEG150-C18, methanol | 1.25 wt% PVP 55KD | 340 | 37 | |
| 14 | Danazol | PEG150-diC18, methanol | 1.25 wt% PVP 55KD | 355 | 107 | |
| 15 | Danazol | Tween 20, methanol | 1.25 wt% PVP 55KD | 350 | 17 | |
| 16 | Danazol | Span 20, methanol | 1.25 wt% PVP 55KD | 407 | 79 | |
| 17 | Danazol | F-108, acetone | 1.25 wt% PVP 55KD | 358 | 89 | |
| 18 | Danazol | F-108, acetone | 2.5 wt% PVP 29KD | 211 | 185 | |
| 19 | Danazol | F-108, acetone | 2.5 wt% PVP K-60 | 303 | 170 | |
| 20 | Danazol | F-108, acetone | 2.5 wt% PVA 31-50KD | 196 | 102 | |
| 21 | Danazol | F-88, acetic acid | 1.25 wt% PVP 55KD | 326 | 44 | |

### Comparative Examples 22 through 24: Drug particles as received.

Particles of Danazol, Naproxen, and Carbamazepine as received from the supply companies were analyzed using X-ray diffraction. To determine the dissolution rates, 3-5 mg of samples containing the drugs Danazol, Naproxen, or Carbamazepine as received were dispersed exactly as described above for Examples 1 through 21. Dissolution was monitored for 5 minutes by the loss in turbidity. Table B below lists the materials used and the results.

### Comparative Examples 25 through 26: Drug particles prepared using a wet milling process.

1.35 g of a stabilizer as indicated in Table B was dissolved in 12 g of water and placed in a wide mouth jar. 1.35 g of the drug powder and 100 g of 1-mm zirconium oxide milling beads were added to this mixture. The jar was then placed on a rotating ball mill and milled for the length of time indicated in Table B. The Jar was removed, the milling beads filtered off, and the resulting slurry spray dried to a powder. Dissolution was monitored for 5 minutes by the loss in turbidity. Table B below lists the materials used and the results.

**Table B**

| **Ex.** | **Drug** | **Preparation** | **Aqueous soln** | **Crystalline Domain [Å]** | **Percent of Dissolution after 5 min.** |
|---|---|---|---|---|---|
| 22 (Comp.) | Naproxen | as received | | 646 | 72 |
| 23 (Comp.) | Carbamazepine | as received | | 475 | 100 |
| 24 (Comp.) | Danazol/USP 24 | as received | | 404 | 91 |
| 25 (Comp.) | Naproxen | wet-milled/ 8 hours | F-68 | 508 | 94 |
| 26 (Comp.) | Danazol | wet-milled/ 1 hour | F-127 | 349 | 97 |

## Claims

1. A process for preparing drug particles, comprising the steps of :
(a) dissolving a drug substance in a solvent; and
(b) adding the product of step (a) to water to form precipitated drug particles; wherein a stabilizer is initially present in the solvent and/or in the water,
wherein the drug particles comprise:
a plurality of crystalline domains, each domain being oriented differently than any of the adjacent domains, wherein the domains comprise a drug substance; and
a plurality of interfacial regions surrounding the crystalline domains, the interfacial regions comprising at least one stabilizer.

2. The process according to Claim 1 wherein the average size of the crystalline domains is less than about 500 Angstroms.

3. The process according to Claim 1 wherein the stabilizer is one or more phospholipids, surfactants, vesicles, polymers, copolymers, homopolymers, biopolymers, or dispersion aids.

4. The process according to Claim 1 wherein the particles are essentially crystalline.

5. The process according to Claim 1 wherein the drug substance is poorly soluble in water.

6. The process according to Claim 5 wherein the drug substance is intended for oral administration.

7. The process according to Claim 1 further comprising the step of mixing the product of step (b).

8. The process according to Claim 1 further comprising the step of drying the precipitated drug particles.

9. The process according to Claim 1, wherein step (b) is performed at less than about 65°C.

10. The process according to Claim 1, wherein one or more excipients is present in the solvent or in the water.

## Patentansprüche

1. Verfahren zur Herstellung von Arzneipartikeln, umfassend die Schritte:
(a) Auflösen einer Arzneisubstanz in einem Lösungsmittel und
(b) Hinzufügen des Produkts aus Schritt (a) zu Wasser, um präzipitierte Arzneipartikel zu bilden,
wobei ein Stabilisator anfangs in dem Lösungsmittel und/oder in dem Wasser vorhanden ist,
wobei die Arzneipartikel umfassen:
mehrere kristalline Domänen, von denen jede Domäne anders ausgerichtet ist als jede der benachbarten Domänen, wobei die Domänen eine Arzneisubstanz umfassen, und
mehrere Grenzflächenbereiche, welche die kristallinen Domänen umgeben, wobei die Grenzflächenbereiche wenigstens einen Stabilisator umfassen.

2. Verfahren nach Anspruch 1, wobei die durchschnittliche Größe der kristallinen Domänen weniger als etwa 500 Angström beträgt.

3. Verfahren nach Anspruch 1, wobei der Stabilisator ein oder mehrere Phospholipide, oberflächenaktive Mittel, Vesikel, Polymere, Copolymere, Homopolymere, Biopolymere oder Dispergierungshilfsmittel ist.

4. Verfahren nach Anspruch 1, wobei die Partikel im Wesentlichen kristallin sind.

5. Verfahren nach Anspruch 1, wobei die Arzneisubstanz in Wasser schwach löslich ist.

6. Verfahren nach Anspruch 5, wobei die Arzneisubstanz für die orale Verabreichung bestimmt ist.

7. Verfahren nach Anspruch 1, weiter umfassend den Schritt des Vermischens des Produkts aus Schritt (b).

8. Verfahren nach Anspruch 1, weiter umfassend den Schritt des Trocknens der präzipitierten Arzneipartikel.

9. Verfahren nach Anspruch 1, wobei Schritt (b) bei weniger als etwa 65°C durchgeführt wird.

10. Verfahren nach Anspruch 1. wobei ein oder mehrere Hilfsstoffe in dem Lösungsmittel oder in dem Wasser enthalten sind.

## Revendications

1. Procédé pour préparer des particules médicamenteuses, comprenant les étapes de :
(a) dissoudre une substance médicamenteuse dans un solvant ; et
(b) ajouter le produit de l'étape (a) à de l'eau pour former des particules médicamenteuses précipitées ;
où un stabilisant est présent initialement dans le solvant et/ou dans l'eau,
où les particules médicamenteuses comprennent :
une pluralité de domaines cristallins, chaque domaine étant orienté différemment de l'un quelconque des domaines adjacents, où les domaines comprennent une substance médicamenteuse ; et
une pluralité de régions interfaciales entourant les domaines cristallins, les régions interfaciales comprenant au moins un stabilisant.

2. Procédé selon la revendication 1 où la taille moyenne des domaines cristallins est inférieure à environ 500 Angströms.

3. Procédé selon la revendication 1 où le stabilisant est un ou plusieurs phospholipides, tensioactifs, vésicules, polymères, copolymères, homopolymères, biopolymères ou adjuvants de dispersion.

4. Procédé selon la revendication 1 où les particules sont essentiellement cristallines.

5. Procédé selon la revendication 1 où la substance médicamenteuse est peu soluble dans l'eau.

6. Procédé selon la revendication 5 où la substance médicamenteuse est destinée à l'administration orale.

7. Procédé selon la revendication 1 comprenant en outre l'étape de mélange du produit de l'étape (b).

8. Procédé selon la revendication 1 comprenant en outre l'étape de séchage des particules médicamenteuses précipitées.

9. Procédé selon la revendication 1 où l'étape (b) est accomplie à moins d'environ 65°C.

10. Procédé selon la revendication 1 où un ou plusieurs excipients sont présents dans le solvant ou dans l'eau.
